(19) Europäisches Patentamt
European Patent Office
Office européen des brevets

(11) EP 0 994 901 B1

(12) **FASCICULE DE BREVET EUROPEEN**

(45) Date de publication et mention de la délivrance du brevet:
**27.10.2004 Bulletin 2004/44**

(21) Numéro de dépôt: **99913402.6**

(22) Date de dépôt: **14.04.1999**

(51) Int Cl.[7]: **C07K 14/435**, A61K 38/17, A61K 7/00

(86) Numéro de dépôt international:
**PCT/FR1999/000866**

(87) Numéro de publication internationale:
**WO 1999/052940 (21.10.1999 Gazette 1999/42)**

(54) **EXTRACTION, IDENTIFICATION, UTILISATION, DES PRINCIPES ACTIFS DES COQUILLES DES MOLLUSQUES MARINS**

EXTRAKTION, IDENTIFIZIERUNG UND VERWENDUNG VON MUSCHELWIRKSTOFFEN MARITIMER MOLLUSKEN

METHOD FOR EXTRACTING, IDENTIFYING, USING ACTIVE PRINCIPLES OF MARINE MOLLUSQUE SHELLS

(84) Etats contractants désignés:
**AT BE CH CY DE DK ES FI FR GB GR IE IT LI LU MC NL PT SE**

(30) Priorité: **14.04.1998 FR 9804635**

(43) Date de publication de la demande:
**26.04.2000 Bulletin 2000/17**

(73) Titulaires:
- **Camprasse, Serge**
  **75019 Paris (FR)**
- **Camprasse, Georges**
  **77480 Villenauxe-la-Petite (FR)**

(72) Inventeurs:
- **Camprasse, Serge**
  **75019 Paris (FR)**
- **Camprasse, Georges**
  **77480 Villenauxe-la-Petite (FR)**

(74) Mandataire: **Poupon, Michel**
**Cabinet Michel Poupon,**
**3 rue Ferdinand Brunot**
**88026 Epinal Cedex (FR)**

(56) Documents cités:
**WO-A-95/30426** **WO-A-97/24133**

- **J. KEITH ET AL.: "Comparative analysis of macromolecules in mollusc shells" COMPARATIVE BIOCHEM. AND PHYSIOL., vol. 105B, no. 3-4, 1993, pages 487-496, XP002089924**
- **PATENT ABSTRACTS OF JAPAN vol. 012, no. 084 (C-482), 17 mars 1988 (1988-03-17) & JP 62 223104 A (MIKIMOTO SEIYAKU KK), 1 octobre 1987 (1987-10-01)**
- **PATENT ABSTRACTS OF JAPAN vol. 018, no. 601 (C-1274), 16 novembre 1994 (1994-11-16) & JP 06 228003 A (MIKIMOTO PHARMACEUT CO LTD), 16 août 1994 (1994-08-16)**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

EP 0 994 901 B1

Printed by Jouve, 75001 PARIS (FR)

## Description

La présente invention concerne un procédé d'extraction par hydrolyse à froid, de séparation et d'identification, des principes actifs protéiques et non protéiques présents dans la coquille de mollusques marins gastéropodes à savoir le Tridacne Bénitier et la Pinctada Maxima et leur utilisation dans des préparations entrant dans la composition de substances médicamenteuses, de milieux de diagnostics et de formulations cosmétiques.

On sait que la coquille des mollusques est composée de deux types ultra-structuraux qui sont des polymorphes du carbonate de calcium $CaCo_3$. Ces deux polymorphes sont la calcite et l'aragonite. La calcite constitue la partie externe de la coquille et est composée de cristaux qui cristallisent dans le système cristallin rhomboédrique, tandis que l'aragonite encore appelée nacre, constitue la partie médiane et interne dont les cristaux cristallisent dans le système cristallin orthorhombique. Les biocristaux des parties internes et externes sont entourés de feuillets et de cloisons de matière organique.

Il est déjà connu, des documents WO 97 24133, WO 95 30426 et J. KEITH ET AL d'extraire les principes actifs de la nacre d'un mollusque, la coquille externe étant systématiquement éliminée.

Aucun document ne propose l'utilisation de la coquille entière du mollusque et donc l'extraction de tous les composants de haut poids moléculaire.

On a mis en évidence dans la matrice organique la présence de constituants protéiques et non protéiques, mais les méthodes d'investigations classiques ne permettaient pas de caractériser tous les constituants notamment ceux de haut poids moléculaire, d'autant que ces méthodes utilisent l'hydrolyse à chaud par des acides forts ou l'hydrolyse à température ambiante par des acides faibles, ce qui provoque, non seulement la rupture des liaisons covalentes des substances protéiniques étudiées, mais encore dénaturent les protéines et altèrent leurs structures primaire et tertiaire initiales provoquant une perte de leur activité chimique et biologique. Il fallait donc trouver un procédé permettant non seulement d'extraire et de caractériser les aminoacides, les protéines, mais encore les glycoprotéines, les glycosaminoglycanes et les protéoglycanes et de les intégrer dans des préparations à visée thérapeutique, diagnostic et cosmétique.

Pour la préparation de 100 kg de poudre de coquille externe et interne, on commence par obtenir par découpage des fragments de coquilles. Dans l'exemple selon l'invention, on prend séparément 100 kg de coquilles de Tridacne Bénitier et de Pinctada Maxima, coupées afin d'obtenir des morceaux de 5 cm de longueur, sur 3 cm de largeur. Ces fragments après lavage sous pression de 3 bars, sont traités par ultra sons pendant 5 mn, sans détergent, et décontaminés par immersion dans une solution de calbénium pendant 1 heure, puis sont placés dans des bols en zirconium de 10 cm de diamètre et de profondeur, dans lesquels sont placées 6 billes en zirconium, de 20 mm de diamètre. Les bols sont ensuite placés dans un broyeur planétaire, le dispositif tournant alternativement dans le sens des aiguilles d'une montre et inversement, à une vitesse de rotation de 1500 tour/mn pendant deux phases de 3mn jusqu'à l'obtention d'une poudre dont la granulométrie se situe entre 300 et 500 microns.

Le mode opératoire du procédé d'extraction selon l'invention, consiste à placer dans un récipient en matériau plastique inerte, d'une contenance de 1100 litres, 300 1 d'eau (soit 58%) apyrogène, 80 kg de glace (13%) grossièrement broyée, obtenue à partir de la même eau, 150 l d'acide acétique (29%) à 80%, le mélange est constamment agité par un agitateur du commerce de puissance suffisante pour 50 à 1000 litres. Un pH mètre contrôle en permanence le pH de la solution qui doit être toujours inférieur à 4,5, un thermomètre du commerce contrôle la température qui doit rester inférieure à 5°C et plus souvent voisine de 0°C.

Dans cette préparation est versée de manière très lente, par fraction de 20 kg toutes les trois heures, la poudre de coquille, tout en contrôlant la production de mousse qui peut être recueillie dans un récipient de garde.

Un dispositif composé d'un circuit fermé de tuyaux en plastique dans lequel circule à vitesse réduite, du polyéthylène glycol, servant de réfrigérant à la préparation est placé à l'intérieur du récipient de 1100 l.

De la glace broyée est régulièrement ajoutée à la préparation afin d'avoir une température inférieure à 5°C et plus souvent voisine de 0°C, de la même manière le pH mètre est couplé à une électrovanne qui permet l'addition de l'acide acétique, dès que le pH augmente. On obtient ainsi dans le récipient, la réaction suivante :

$$\text{acide acétique + carbonate de calcium} \rightarrow \text{acétate de calcium + eau}$$

$$CH_3\,COOH + CaCO_3 \rightarrow CO_2 + H_2O + Ca\,(CH_3\text{-}COO)_2$$

Une fois dissous les 100 kg de poudre de coquilles, la totalité de la solution est filtrée sur un tamis, puis centrifugée dans une super centrifugeuse (Sharpless, bol de 6 litres) à une force centrifuge de 18 000 G. On obtient sur les parois du bol un culot (10kg) de substances organiques humides qui est réservé dans un récipient stérile, inerte et hermétique. On centrifuge une deuxième fois le surnageant afin de le clarifier. On obtient un second culot de centrifugation, les deux culots, réunis, sont lavés de la façon suivante :

- dans une bassine en matériau inerte d'une contenance de 100 l, pourvue du même dispositif de refroidissement par un circuit interne fermé de polyéthylène glycol, la bassine étant placée dans un récipient de garde rempli de glace pilée renouvelée selon les besoins, ceci afin de maintenir le bain à une température inférieure à 5° C et plus souvent voisine de 0°C, on place les deux culots de centrifugation, auxquels on ajoute 65 l d'acide acétique à 5%, la solution est centrifugée, dans une super centrifugeuse à une force de 18 000 G, on obtient un culot qui est repris par 65 1 d'eau apyrogène. Le pH de la solution est amené à pH 7 par adjonction de soude 2,5 N, le tout étant agité constamment par un agitateur pendant 1 heure, puis centrifugé à 18000 G. Le culot ainsi obtenu est repris dans 65 litres d'eau apyrogène sous agitation constante puis à nouveau centrifugé. On obtient un culot définitif de matière organique humide qui est séché par lyophilisation à froid. On prélève 10cc des eaux du dernier lavage qui sont traitées par l'acide oxalique afin de vérifier la présence ou l'absence de calcium par la réaction suivante :

$$\text{acétate de calcium + acide oxalique} \rightarrow \text{oxalate de calcium + eau}$$

Après séchage le produit selon le procédé se présente sous la forme de poudre grossière de couleur gris foncé d'un poids (de 1.8 kg pour la Pinctada, et 2.3 kg pour le Tridacne Bénitier) qui est finement broyée au mortier afin d'obtenir une poudre de granulométrie variant entre 0,5 et 2 microns.

La matière organique ainsi obtenue contient les constituants organiques insolubles contenus dans la coquille du Tridacne, Bénitier ou de la Pinctada Maxima,

On reprend la totalité des surnageants récupérés lors de l'extraction des constituants protéiques insolubles, l'ensemble contenant les constituants protéiques solubles et les sels de calcium sous la forme acétate, c'est ainsi que l'on a 800 litres de solution.

Après passage au travers d'un filtre en tissu, les surnageants sont dessalés par passage dans une batterie de 17 cassettes d'osmoses tangentielles de lkdalton de 0,5 m2 chacune, montées en série, sous une pression de 9 bars. Le débit de dessalage est de 50 à 70 ml par mn par cassette, soit pour 17 cassettes 1 litre par minute. Lorsqu'il reste 50 litres de solution salée, on dilue par 50 litres d'eau apyrogène dans un récipient inerte stérile, d'une contenance de 150 litres, refroidie par les mêmes dispositifs et procédés décrits plus haut. On effectue ensuite une série de 6 dilutions, ultra filtration avec chaque fois 50 litres d'eau apyrogène. Au cours de l'avant dernière dilution, on amène le pH à pH 7 par de la soude 2,5N. Après plusieurs ultra filtrations successives suivis de rinçages, on obtient environ 20 l de solution que l'on concentre à 5 l au Rotavapor sous vide à moins de 40 °C. La solution est ensuite lyophilisée, l'extrait est ensuite broyé au mortier jusqu'à l'obtention d'une granulométrie comprise entre 0.2 et 0.5 microns. On obtient ainsi une poudre allant du blanc au blanc grisâtre, représentant la fraction soluble des constituants protéiques de la coquille du Tridacne Bénitier (328g) ou de Pinctada Maxima(100g).

A la fin du procédé selon l'invention, on vérifie dans les surnageants, la présence ou l'absence de protéines en prélevant une aliquote de solution qui est traitée par la méthode colorimétrique de Biuret (coloration bleue en présence de protéines, apparaissant rapidement et restant stable pendant plus de 2 heures). L'intensité de cette coloration variant linéairement avec la concentration en protéines. L'absence de coloration signifie que toutes les protéines ont été extraites. Les surnageants sont aussi traités par la méthode de

Lowry, qui est plus sensible mais plus longue. Une des variantes de cette méthode consiste à précipiter quantitativement dans un premier temps, les protéines par mélange avec trichloracétique ou désoxycholate de sodium avant de procéder à la coloration. L'absence de coloration montre que toutes les protéines ont été extraites. Ces deux méthodes ont l'avantage d'être relativement rapides et fiables, mais ne rendent compte que de la présence ou non de protéines dans le surnageant.

Les deux fractions organiques ainsi recueillies sont placées dans des récipients stériles et stérilisés par rayonnement Gamma 2.5 mégarad et conservées à une température de 3°C.

L'hydrolyse à froid, la centrifugation et l'ultracentrifugation sur cassettes en série d'après le procédé suivant l'invention, nous ont permis d'extraire les constituants organiques insolubles et solubles de la coquille des bivalves étudiés. Les différentes composantes organiques seront caractérisées qualitativement et quantitativement par les méthodes analytiques habituelles qui seront utilisées soit séparément soit en association. Ces méthodes sont la chromatographie analytique par échange ionique, qui permettent par réaction et dérivation de prélever des aliquotes des fractions de l'effluent et de leur faire subir une réaction spécifique, par exemple pour les acides aminés une réaction à la ninhydrine qui provoque une coloration que l'on peut mesurer à l'aide d'un spectrophotomètre. Cette technique utilise des colonnes de chromatographie comportant des substances hautement polymérisées constituant un réseau tridimensionnel et portant des fonctions acides ou basiques. Ces substances servent de support aux groupements chimiques chargés de réaliser cet échange.

Ces substances sont des résines ou des dérivés de la cellulose ou des polymères glucidiques. On utilise

aussi pour la séparation des acides aminés et des oses, la chromatographie sur couches minces dont le principe fait appel au fractionnement par partage entre deux solvants, un autre mode de séparation utilisé est la chromatographie sous haute pression (plus de 30 Bars) qui utilise la méthode de chromatographie en phase inverse adaptée à la séparation de molécules de faible poids moléculaire, et aussi à celle des protéines qui sont des macromolécules. Ce type de chromatographie à haute pression utilise aussi des colonnes d'échanges d'ions pour la séparation d'acides aminés. On associe à la chromatographie l'électrophorèse qui permet aussi la séparation des protéines. La révélation de la présence de protéines est réalisée grâce à des colorants (noir amidé, rouge de Ponceau), il existe aussi des colorants plus spécifiques comme le réactif de Schiff qui révèlent les fractions glucidiques des protéines. L'électrophorèse de zone sur gel de polyacrylamide qui permet une séparation selon la charge et le poids moléculaire, l'utilisation d'un gel de polyacrylamide en présence de dodécylsulfate de sodium (SDS), associé à un traitement au mercaptoéthanol préalable à la migration de la substance protéique à séparer, permet de déterminer le poids moléculaire, et le nombre de chaînes polypeptidiques composant la protéine. On a déjà, par hydrolyse acide (HCl) à chaud, isolé plusieurs acides aminés, exactement 17.

Ces méthodes d'identification appliquées à la fraction soluble des constituants extraits du Tridacne Bénitier et de la Pinctada Maxima, a permis de mettre en évidence, les acides aminés suivants, avec leur pourcentage en moles :

| moles % | | |
|---|---|---|
| Pinctada Maxima | | Tridacne Bénitier |
| Asp | 11,8 | 15,2 |
| Thr | 1,12 | 3,5 |
| Ser | 4,10 | 7,3 |
| Glu | 2,58 | 6,15 |
| Pro | 1,14 | 3,64 |
| Gly | 28, 95 | 32,95 |
| Ala | 29,27 | 33,27 |
| Cys/2 | 0,71 | 3,71 |
| Val | 8,02 | 11,06 |
| Met | 1,07 | 3,15 |
| Ile | 1,16 | 3,72 |
| Leu | 6,42 | 9,65 |
| Tyr | 1,99 | 4,25 |
| Phe | 1,68 | 4,10 |
| His | 0,15 | 3,12 |
| Lys | 1,57 | 3,86 |
| Arg | 4,23 | 7,66 |

La mise en évidence qualitative et quantitative du tryptophane soit 3,95 moles % est obtenue par hydrolyse enzymatique par la trypsine brute de 100 g de poudre de coquille interne et externe de Pinctada Maxima et de Tridacne Bénitier, dont la granulométrie est de 1 micron. L'hydrolysat ainsi obtenu est ensuite traité par l'acide glyoxylique en milieu sulfurique, la coloration violette (réaction d'Adamkiewicz), caractérise la présence de tryptophane, confirmée également par la réaction d'EHRLICH (coloration bleue en présence de p-diméthylaminobenzaldéhyde en milieu acide).

La présence et l'analyse quantitative de certains acides aminés nous ont permis par analogie de mettre en évidence, la présence de protéines dont ils sont constitutifs, bien que ces acides aminés existent à l'état libre. C'est ainsi que le pourcentage élevé de glycocolle (28,95) laisse supposer la présence confirmée de scléroprotéines comme les collagènes de type I - III - IV ou l'élastine. La proportion élevée d'Alanine (Ala) montre qu'elle existe aussi bien à l'état libre, que comme constituant d'une protéine. L'acide aspartique et glutamique sont des constituants importants des protéines. La Lysine, l'Arginine, et l'Histidine sont les acides aminés des protéines. La Lysine de formule générale

$$N_2H - (CH_2)_4 - CH \begin{matrix} \nearrow COOH \\ \searrow NH_2 \end{matrix}$$

peut être rattachée à l'acide alpha gamma diaminobutyrique qui est un constituant de certains antibiotiques peptidiques de formule générale

$$H_2N - (CH_2)_2 - CH \begin{matrix} \nearrow COOH \\ \searrow NH_2 \end{matrix}$$

qui en fait un homologue inférieur de la lysine.

Cette similitude de formule générale explique certaines propriétés antibiomimétiques des constituants des composants organiques extraits de la coquille externe et interne du Bénitier et de la Pinctada Maxima par le procédé selon l'invention et la mise en évidence de la S-hydroxylysine signe la présence de collagènes de même que celle de la Proline.

S'il est facile d'identifier, par les méthodes énumérées plus haut, les acides aminés et les protéines, il est plus difficile d'identifier la présence de glycoprotéines. Les glycoprotéines sont des hétéroprotéines résultant de l'union d'une fraction protéinique avec une ou plusieurs séquences glucidiques, elles sont stables à la chaleur, solubles dans les solutions salines au voisinage de la neutralité. Ce sont des macromolécules d'un poids moléculaire très élevé. La teneur en glucides de ces macromolécules est très variable de 1% à plus de 40%.

Le problème de l'arrangement séquentiel des glucides dans les chaînes glucidiques des glycoprotéines rend très difficile la résolution de ces séquences, pour cela nous avons employé une combinaison de plusieurs techniques : chimiques et enzymatiques. La liaison glucide - polypeptide apparaît dans la majorité des cas au niveau des résidus Séryl et thréonyl, d'une part, et de résidus asparaginyl, d'autre part, de la chaîne polypeptidique. Nous avons utilisé les méthodes suivantes : la méthylation par l'iodine ou le sulfate de méthyle, l'acétolyse en milieu anhydre entraînant une coupure des liaisons osidiques, suivie d'une saponification ; le protocole de la dégradation séquentielle de Smith, l'hydrolyse acide en corrélation avec d'autres méthodes, l'hydrolyse enzymatique dans le méthanol anhydre, l'hydrolyse enzymatique par une aspartylglucosanimidase et par des hexosanimidases, toutes ces méthodes demandent des systèmes enzymatiques très purs.

Elles ont mis en évidence la présence de glycoprotéines dans les constituants extraits de la fraction organique des coquilles internes et externes du Tridacne Bénitier et de la Pinctada Maxima, mais celles-ci n'ont pas subi d'analyse séquentielle permettant de les comparer aux modèles glycoprotéiniques déjà connus. On sait cependant que les glycoprotéines sont impliquées dans le processus d'adhésivité et de reconnaissance cellulaire et que celles mises en évidence dans les constituants organiques des coquilles du Tridacne Bénitier et de la Pinctada Maxima, proviennent de la membrane des cellules du manteau qui sont des fibroblastes, et qui fabriquent les couches internes et externes des coquilles, elles proviennent aussi du tissu conjonctif, du manteau, et de la matrice extra-cellulaire que l'on retrouve dans le liquide extra-palléal et qui contient une très forte concentration de macromolécules nécessaires à l'édification de la coquille de ces mollusques. La réaction de Maillard indique nettement la présence des glycoprotéines contenues dans les coquilles internes et externes des mollusques, tels que le Tridacne Bénitier et de la Pinctada Maxima. Cette réaction consiste à chauffer très fortement en milieu anhydre, 100 g de constituants extrait par le procédé selon l'invention : les oses présents dans les protéines réagissent avec les fonctions amines des protéines solubles ($-NH_2$ de la lysine, qui entre dans leur composition). Il se forme un résidu brun très foncé, résultat de la condensation du ou des sucres contenus dans la glycoprotéine et les fonctions amines des protéines avec un remaniement de type Amadori, conduisant à des produits décarboxylés et une polymérisation en composés bruns.

L'utilisation de réactifs colorés ainsi que l'utilisation d'enzymes spécifiques permettent de mettre en évidence dans la coquille interne et externe des mollusques cités plus haut, des macromolécules particulières que sont les glycosaminoglycanes (GAG) terme générique qui désigne des polysaccharides acides constitués par des chaînes linéaires de résidus régulièrement alternés de N-acétyl hexosamine et d'acide hexuronique. Ces mucopolysaccharides sont par leurs structures proches de l'acide hyaluronique, car ils sont présents dans le tissu conjonctif du manteau où ils constituent comme l'acide hyaluronique, une espèce de ciment intercellulaire, dont la fonction capitale est de s'opposer à la diffusion des substances étrangères, comme par exemple, les agents pathogènes. Nous avons utilisé pour

la mise en évidence des glycosaminoglycanes contenus dans la coquille interne et externe des mollusques cités plus haut, les techniques histochimiques et immunologiques ; notamment la coloration histochimique par le rouge de ruthénium, l'acide phosphotungstique qui provoque un précipité noir en présence de glycosaminoglycanes ainsi que l'examen au microscope électronique des extraits de la coquille interne et externe des mollusques étudiés après incubation dans un milieu contenant des anticorps spécifiques qui montre un résidu filamenteux correspondant au complexe antigène anticorps. C'est ainsi que l'on peut expliquer expérimentalement et par observation directe, que certains agents pathogènes comme les vibrios, se retrouvent bloqués soit dans l'épaisseur de la coquille interne, soit dans le tissu conjonctif du mollusque, et qu'ainsi il y a inhibition de la diffusion des toxines libérées par cet agent pathogène. De la même façon la mise en évidence d'hexosamines sulfatées, signe la présence de glycosaminoglycanes voisins de la chondroitine sulfate, dont l'importance est capitale dans le processus de formation de la coquille en cours d'édification (fixation de cations comme le calcium).

On connaît l'importance de l'acide hyaluronique qui est le plus simple des glycosaminoglycanes et qui provient directement de la surface cellulaire. On pense que l'acide hyaluronique joue un rôle dans la résistance des tissus aux forces de compression. Sa présence dans la composition des coquilles externes et internes des mollusques étudiés par le procédé selon l'invention, explique la valeur du module d'élasticité en compression (90 000 N/$mm^2$) de la coquille nacrée, tel qu'il a pu être défini par le Laboratoire National d'Essais. C'est aussi un glycosaminoglycane très hydrophile, en effet une petite quantité de GAG peut s'étendre et occuper un grand volume. L'acide hyaluronique est généralement produit en grande quantité au cours de la cicatrisation et sert de lubrifiant dans les articulations. Sa présence a pu être mise en évidence expérimentalement lors de la perforation de la coquille des mollusques étudiés tels que le Tridacne Bénitier et la Pinctada Maxima : durant le processus de réparation de la perte de substance, on note l'apparition du tissu cicatriciel très précoce à forte composante organique, dont l'étude analytique a montré, outre la présence de scléroprotéines ou protéines fibreuses, de glycosaminoglycanes sulfatés et d'acide hyaluronique.

A l'exception de l'acide hyaluronique qui est un glycosaminoglycane simple, il existe des glycosaminoglycanes qui se lient à des protéines pour former des protéoglycanes. Les protéoglycanes se distinguent des autres protéines par leur nature, la quantité et l'agencement de leur chaîne glucidique latérale. Les protéoglycanes sont d'énormes édifices plurimoléculaires dont les noyaux protéiques, vont de 10 000 à plus 600 000 Daltons. Les protéoglycanes sont en fait des glycoprotéines très fortement glycosilées, leur identification et leur classification est très difficile à mettre en évidence. Cependant elle est rendue possible indirectement par l'utilisation des phytohémagglutinines qui se fixent sur les glucides spécifiques portés par les molécules protéiques formant une volumineuse molécule facilement visible au microscope électronique. On pense que les protéoglycanes jouent un rôle important dans la signalisation chimique intercellulaire, ils sont capables de se lier in vivo et in vitro à des molécules informatives, tels que les facteurs de croissance protéiques ; ils peuvent contrôler l'activité d'autres types de protéines sécrétées tels les enzymes, ils peuvent également s'associer au collagène. Les protéoglycanes font partie de la fraction soluble des composants organiques de la coquille interne et externe du Tridacne Bénitier et de la Pinctada Maxima. La mise en évidence de protéines glycosilées monomériques d'un poids atomique d'environ 40 000 Daltons, que sont les cytokines a été réalisée expérimentalement. Leur action a pu être constatée lors de phénomènes de cicatrisation secondaire à une altération des tissus par brûlure, ce sont pour la plupart des facteurs ou des profacteurs de croissance, dont le rôle consiste à stimuler, potentialiser, réparer induire ou inhiber, certains processus chimiques et biologiques.

Les méthodes habituelles de chromatographie, d'ultracentrifugation ainsi que les tests spécifiques et colorimétriques (test d'Awapara, test de solubilité dans les solvants) de même que les mesures chromatométriques ont permis de mettre en évidence dans les produits d'extractions des coquilles externes et internes des mollusques étudiés, la présence de composés macromoléculaires insolubles d'un poids moléculaire allant de 100 à 10000 Daltons, de la famille des mélanines qui sont issus de la tyrosine par oxydation et qui donne à la fraction insoluble des constituants organiques une coloration allant du brun au noir. L'analyse qualitative et quantitative des minéraux et des métaux contenus dans la coquille des mollusques, telle que Pinctada Maxima, a déjà été réalisée. Les procédés d'analyse identique appliqués au Tridacne Bénitier par le procédé selon l'invention, ont montré la présence de ces mêmes éléments minéraux et métalliques, que sont le Sémarium (Sm) à l'état de trace, le Lutérium (La), le Zinc (Zn), le Brome (Br), le Cérium (Ce), le Fer (Fe), le Manganèse (Mn), le Chlore (Cl), le Potassium (K), le Strontium (Sr), le Sodium (Na), le Calcium (Ca). Certains atomes de métaux constituent des oligo-éléments, d'autres sont associés à des protéines pour former des métalloprotéines non enzymatiques ou le métal intervient comme forme de transport ou de réserve, c'est le cas du fer, du cuivre qui est associé à une protéine, pour former une métalloprotéine servant de pigment respiratoire chez le mollusque, c'est le cas du zinc, qui peut être pris également comme un oligo-élément ou associé à une protéine pour former aussi une métalloprotéine, à laquelle on reconnaît une fonction régulatrice dans l'expression des gènes.

Le calcium qui est l'élément minéral de la coquille, peut aussi former une calciprotéine, dont le rôle est de régler l'activité enzymatique. Les éléments métalliques sont également associés à une protéine enzymatique pour former des métalloenzymes, c'est le cas du manganèse, du fer et du zinc. Ces métalloenzymes interviennent dans le mécanisme de destruction des superoxydes ($O_2$-)

D'ailleurs les métaux et minéraux qui existent à l'état libre ou pouvant être associés à des protéines ou à des enzymes sont ceux dont la concentration est la plus élevée.

Les méthodes chromatographiques associées à des études de fluorescence ont également mis en évidence des pigments autres que les composés mélaniniques, qui sont des chromoprotéines, aussi bien des chromoprotéines porphyriniques contenant des pigments respiratoires et non respiratoires, que des chromoprotéines non porphyriniques contenant les mêmes pigments. On peut rattacher ces chromoprotéines aux métalloprotéines déjà caractérisées. C'est ainsi que l'on a mis en évidence, outre les porphyrines, des métalloporphyrines et des caroténoides dont on retrouve les teintes dans la nacre des perles et dans le test nacré.

La principale propriété chimique des porphyrines est leurs aptitudes à se combiner aux métaux, surtout ceux de la famille du fer (fer, chrome, manganèse, nickel, cobalt) au cuivre, au zinc pour donner naissance à des complexes non ionisables, que sont les métalloporphyrines, dont les propriétés sont semblables à celles des métalloenzymes et des métalloprotéines non enzymatiques. L'extraction des lipides du complexe organominéral des coquilles externes et internes des mollusques étudiés a été réalisée par saponification de 100g d'extraits solubles et insolubles de coquilles des mollusques étudiés, à l'aide d'un excès de solution alcoolique de potasse portée à ébullition dans un tube à essai, l'addition d'éther de pétrole permet de soutirer une phase hydroalcoolique basique qui est acidifiée, ce qui fait précipiter les acides gras, ceux ci peuvent être recueillis dans l'éther de pétrole par évaporation. Cette extraction a permis de mettre en évidence des substances liposolubles qui sont des caroténoides qui donnent à la coquille interne sa coloration, jaune orangé. Ces caroténoides dont les principaux sont les carotènes de formule générale $C_{40}H_{56}$ dont on connaît 3 isomères, sont des provitamines A

L'importance des éléments constitutifs déjà connus, des coquilles internes et externes des mollusques cités dans la description, et ceux caractérisés selon le procédé, la connaissance de leurs propriétés chimiques, biologiques, pharmacodynamiques, permettent la mise en oeuvre de préparations à visée thérapeutique, diagnostic et cosmétique tels que les exemples suivants qui font également l'objet de l'invention sans pour autant être limitatifs.

A titre d'exemple, on trouvera ci-après quelques formulations de base préparées à partir d'extraits des principes actifs solubles et insolubles obtenus selon le procédé.

Exemple 1 - Formulation pour une préparation à visée curative pour brûlures de 1er, 2ème et 3ème degré.

| | |
|---|---|
| Extraits secs de principes actifs solubles | 0,5g |
| Extraits secs de principes actifs insolubles | 0,5g |
| Vitamine C | 1 g |
| Vitamine $B_6$ | 0,5 g |
| Hydroxyde de calcium | 0,5 g |
| Glucose | 4 mg |
| Coprah | 0,5g |
| Carbonate de calcium | 1g |
| Zinc | 14,2 µg |
| Cuivre | 15 µg |
| Sodium | 5 µg |
| Potassium | 7 µg |
| Manganèse | 12,3 µg |
| Fer | 12 µg |
| P Crésol | 0,1 mg |
| Conservateur | |
| Vaseline Q.S. pour | 100g |

Une telle préparation est stérilisée au rayonnement Gamma et conservée dans un emballage stérile.

On sait que lors d'une brûlure, deux types de conséquences doivent être pris en compte pour en apprécier le pronostic vital et fonctionnel : la profondeur et la gravité.

La peau étant un organe à part entière, est de loin le plus étendu et le plus lourd.

Elle est constituée de deux tissus différents mais complémentaires :

l'épiderme qui est la couche externe fine, souple, constituée essentiellement de kératinocytes (97%), mais aussi de mélanocytes et de cellules de Langhérans (responsable du rejet des greffes). Ces cellules sont constituées en

couches qui protègent la peau de la lumière, par la mélanine sécrétée par les mélanocytes et contre les agressions traumatiques par la couche de kératine sécrétée par les kératinocytes.

Le derme sous-jacent constitué d'un réseau de fibres de collagène, élastine et réticuline, de fibronectine, de glycosaminoglycanes et de cément intercellulaire. Ce sont les fibroblastes qui synthétisent les protéines de structure qui le composent. Le derme est innervé et vascularisé par les terminaisons nerveuses et les vaisseaux provenant de l'hypoderme.

L'hypoderme sous-jacent au derme est une couche cellulo-graisseuse.

La réponse physiologique à une agression thermique, se traduit par un grand nombre de perturbations dans quasiment toutes les fonctions de l'organisme, en fonction de la gravité de la brûlure.

Il y a d'abord l'oedème, immédiat, non seulement au niveau de la lésion mais aussi dans les autres tissus en cas de brûlure grave. Les causes de cet oedème sont de trois sortes :

- l'augmentation de la perméabilité capillaire qui est due à la production d'histamine et de médiateurs vasoactifs tels que (sérotonine, bradykinine, prostaglandines, leukotriènes) et à la libération de radicaux oxygénés libres cytotoxiques.
- l'augmentation de l'osmolarité qui est due à la libération d'osmoles intracellulaires par la lésion thermique et à la fixation de molécules de sodium sur les fibres de collagène.
- l'apparition d'une perte plasmatique due à l'évaporation d'autant plus importante que la brûlure est étendue.

Mais les perturbations les plus importantes sont métaboliques. C'est précisément à ce niveau que la préparation selon le procédé est destinée à intervenir.

En effet, dès qu'une lésion atteint 25% de surface brûlée, on assiste à un hypermétabolisme à prédominance catabolique, avec augmentation des dépenses énergétiques, une lipolyse, une hyperglycémie, un hypercatabolisme protidique.

C'est surtout le « turn-over » des protéines qui est augmenté avec une prédominance du catabolisme sur l'anabolisme et transfert des acides aminés depuis les muscles vers les viscères.

On observe une augmentation des cytokines, des TNF. La nécrose tissulaire et la mort cellulaire au niveau du tissu brûlé, provoquent un relargage dans le sang d'un certain nombre de facteurs dépresseurs de l'immunité. JACQUES et coll. et FJELLSTROM et coll. ont montré que le taux de fibronectine est très bas chez les grands brûlés. Presque tous les éléments figurés du sang sont atteints (polynucléaires, lymphocytes, etc.) toutes les protéines de l'inflammation sont augmentées. La disparition ou la baisse des facteurs immunitaires locaux favorisent l'infection. Le traitement de ces perturbations métaboliques est jusqu'à maintenant général. Sur les zones brûlées, on applique un pansement occlusif qui a pour but d'empêcher l'évaporation de l'exsudat et la fuite plasmatique.

La préparation à visée thérapeutique obtenue d'après le procédé à pour but de traiter localement la perte protéique au niveau de la brûlure par apport in situ des acides aminés essentiels de protéines, de protéoglycanes, de glycoprotéines, de glycosaminoglycanes, de sucres, de profacteurs de croissance, d'oligo-éléments et de mélanine, extraits de la coquille interne et externe du mollusque.

L'application de la préparation sur une lésion thermique profonde réalise non seulement un recouvrement cutané, une sédation de la douleur en inhibant la production des produits de l'inflammation (bradykinine, sérotonine, histamine etc.) qui sensibilisent les nocirécepteurs cutanés, mais favorisent aussi la régression de hypermétabolisme. Elle stimule des facteurs locaux de l'immunité cellulaire et tissulaire dont l'action inhibe la prolifération microbienne et partant l'infection.

Nous avons pu également observer un raccourcissement des deux tiers du temps de cicatrisation par augmentation du taux des protéines du stress thermique, qui protègent la synthèse du collagène ; ainsi qu'une régulation du tissu de granulation avec diminution de la taille des bourgeons charnus s'accompagnant d'une augmentation de leur nombre. De plus, la diminution de la mise en tension des fibroblastes empêche de ce fait la formation de cicatrises chéloïdes. Un autre mode de réalisation consiste en une membrane biologique artificielle dont la composition est la suivante : sur un support en treillis de polyglactine d'une épaisseur de 0,5 mm et de dimension de 20 mm sur 20 mm est étendue sur une épaisseur de 0,5 mm, la préparation suivante :

1 mg d'extraits secs des principes actifs solubles et insolubles obtenus selon le procédé
0,5 g de carbonate de calcium
0,1 g d'hydroxyde de calcium
100 mg de vitamine C
25 Uph EUR d'aprotinine (DCI)
thrombine
fibrinogène

La membrane biologique est stérilisée au rayonnement Gamma et conservée au froid à 4°C sous double emballage stérile.

La même formulation à laquelle a été ajouté de l'eugénol a été utilisée sous forme d'onguent pour le traitement du pourrissement de la fourchette du sabot du cheval.

Selon un autre mode de réalisation les produits obtenus selon le procédé peuvent rentrer dans la réalisation d'une préparation pour compléments alimentaires selon une formule qui est la suivante :

- extraits secs de principes actifs solubles 0,1 mg
- extraits secs de principes actifs insolubles 0,1 mg
- vitamine C 200 mg
- vitamine E 1 µg
- vitamine B 2 µg
- cuivre 1 µg
- fer 1 µg
- zinc 1 µg
- amidon Q.S pour 1 g

Les produits obtenus d'après le procédé, peuvent également rentrer dans la composition de préparation à visée cosmétique sous forme de gel ou de crème, dont la composition est la suivante

Extraits secs de principes actifs solubles

- extraits secs de principes actifs insolubles
- eau
- glycérine
- alcool dénaturé
- acide ascorbique
- α-tocophérol
- acide citrique
- extraits de coco
- miel
- propolis
- alginate
- méthyl paraben
- propyl paraben
- excipient QS. pour 100g

La formulation suivante pour applications orthopédiques est préparée à partir des produits obtenus suivant le procédé :

- extraits secs de principes actifs solubles 0,5 g
- extraits secs de principes actifs insolubles 0,5 g
- carbonate de calcium (granulométrie 350 microns) 80 g
- hydroxyde de calcium (granulométrie 10 microns) 10 g
- acide ascorbique 300 mg
- cuivre 0,001 mg
- zinc 0,001 mg
- manganèse 0,001 mg
- vitamine E 0,001 mg

Une telle formulation a été utilisée sous forme de poudre en chirurgie orthopédique humaine et vétérinaire dans 10 cas, où une repousse osseuse était souhaitée. Il s'agissait de fracture avec fracas osseux et perte de substance osseuse.

La préparation ci-dessus a été placée au niveau des pertes de substance sous forme de poudre, mélangée au sang autologue de manière à obtenir une pâte de consistance mastic dans 5 cas. Dans 5 autres cas, elle a été placée sous forme d'éléments prothétiques préformés, de plaques, de pastilles et de tiges. Ces éléments de formes

et de dimensions variables ont été obtenus par moulage de la poudre sous vide, dans un dispositif, sous une pression progressive de 10 tonnes. Un système de refroidissement par un circuit fermé d'éthylène glycol maintenant la température en dessous de 40°C, de manière à ne pas détruire la structure tertiaire de protéines et des autres principes actifs contenus dans la préparation. En effet, lors de la compression la température s'élève de manière constante jusqu'à dépasser 100°C.

Les radiographies postopératoires ont montré dans tous les cas une organisation et une minéralisation du cal de réparation deux fois plus rapide que chez les animaux témoins. Les biopsies pratiquées à 15 jours au niveau du site opératoire, ont mis en évidence une prolifération des ostéoblastes (cellules formatrices d'os), la présence de nombreux capillaires sanguins témoignant d'une angiogénèse active indispensable aux phénomènes de réparation, et une absence d'ostéoclastes (cellules destructrices d'os) qui apparaissent plus tardivement pour remodeler le cal osseux et en harmoniser les contours.

Cette apparition précoce d'ostéoblastes dans le site de réparation et l'absence prolongée d'ostéoclastes, tout au plus au début du processus, prouvent de façon irréfutable la présence de facteurs ou de profacteurs de croissance, qui stimulent l'ostéogénèse, et par conséquent la prolifération des ostéoblastes, et d'autres profacteurs ou facteurs de croissance inhibant la prolifération des ostéoclastes. Cette dernière propriété à été mise en évidence par l'expérimentation suivante : deux cultures d'ostéoclastes ont été réalisées dans deux boites de Pétri en atmosphère stérile et mise en incubation à 37°C. Dans l'une, il a été placé un fragment de coquille de Tridacne Bénitier et un fragment de Pinctada Maxima, ainsi qu'un fragment osseux d'origine animale, sur lequel il a été saupoudré une couche fine d'extraits obtenus d'après le procédé. Dans l'autre boite, a été mis un fragment osseux d'origine animale, seul. Après 3 semaines, l'examen microscopique a montré que dans la première boite, aucun des fragments n'avaient subi d'altération par les ostéoclastes (absence de lacunes de Aoschip), alors que dans la deuxième boite, on a noté, sur le fragment osseux une érosion en coup d'ongle à bords déchiquetés caractéristiques de l'action des ostéoclastes.

Une variante de la formulation précédente, destinée à être injectée au niveau des lésions, est obtenue avec l'adjonction d'un gel à base d'alginate.

Une telle formulation a été utilisée dans une perte de substance du sabot d'un cheval (seime, fourmilière) avec atteinte du bourrelet périoplique (qui assure la repousse de la corne) et mise à nu de la troisième phalange. La préparation selon l'invention est mélangée avec de l'eau déminéralisée à laquelle est ajouté un durcisseur jusqu'à obtention d'un mortier qui est inséré dans la perte de substance. La lésion est progressivement comblée par de la corne néoformée au bout de 5 semaines. Dans un autre cas la préparation selon l'invention à été utilisé lors d'une perte totale du sabot sans atteinte du bourrelet périoplique. La troisième phalange a été entièrement recouverte par la préparation selon l'invention dans un pansement occlusif fixé au tiers inférieur du boulet. La restauration du sabot a été obtenue en 8 semaines alors que dans la plupart des cas, l'animal doit être euthanasié.

Une autre formulation des substances obtenues d'après le procédé, consiste en un milieu de culture.

En effet, la plupart des milieux de culture contiennent des mélanges de macromolécules comme du sérum de cheval ou du sérum de veau foetal, ainsi qu'une quantité précise de petites molécules telles que les sels, le glucose, les acides aminés et les vitamines. De tels milieux sont encore utilisés aujourd'hui pour la plupart des cultures cellulaires de routine, mais ils rendent difficile la détermination précise des macromolécules spécifiques nécessaires au maintien et au fonctionnement normal d'un type cellulaire donné.

Cette difficulté a conduit à la mise au point de divers milieux sans sérum, de composition chimique connue. Ces milieux contiennent différentes protéines spécifiques dont les cellules ont besoin pour survivre et proliférer en culture. Ces protéines incluent les facteurs de croissance qui stimulent la prolifération cellulaire et la transférrine qui transporte le fer aux cellules. De nombreuses molécules informatives extracellulaires, jouant un rôle vital dans le développement et où la prolifération de types cellulaires spécifiques ont été découvertes grâce à l'étude des cellules en culture, et la recherche de nouveaux facteurs est devenue plus aisée depuis que l'on dispose de milieux de composition chimique définie, sans sérum.

La formulation proposée avec les substances obtenues d'après le procédé selon l'invention à la composition suivante :

Extraits secs de principes actifs solubles et insolubles

Dont :

- Asp (25mg/litre) - ClK (50mg/litre)
- Thr (37 mg/litre) - Cu (200mg/litre)
- Ser (27 mg/litre) - Mn (20mg/litre)
- Glu (57 mg/litre) - ClNa (100mg/litre)
- Pro (35mg/litre) - Zn (20mg/litre)
- Gly (40 mg/litre) - Ca (50mg/Litre)
- Ala (2 mg/litre) - Insuline (1mg/litre)
- Cys/2 (75mg/litre) - Transférrine (1mg/litre)

- Val (40mg/litre) - Acide folique (1µg/litre)
- Met (12mg/litre) - Pyridoxal (2µg/litre)
- Ile (48mg/litre) - Thiamine (1µg/Litre)
- Leu (48mg/litre) - Riboflavine (0,2µg/litre)
- Tyr (35 mg/litre) - Vit C (1µg/litre)
- Phe (30mg/litre) - Carbonate de calcium (0,1g/litre)
- His (35m/litre) - Eau apyrogène Q.S pour 1 litre
- Lys (73mg/litre)
- Arg (100mg/litre)

La présence de sérum et de facteurs de croissance n'est pas nécessaire dans un tel milieu, compte tenu du fait, que les produits d'extractions contiennent tous ces éléments. Dans le milieu obtenu d'après le procédé, il a été mis en oeuvre, avec succès, des cultures de cellules osseuses (ostéoblastes, de fibroblastes, de chondroblastes, mélanocytes, de kératinocytes).

## Revendications

1. Procédé d'extraction, de séparation, d'identification, des principes actifs : protéines, glycoprotéines, glycosaminoglycanes et protéglycanes, contenus dans la coquille de Tridacnes Bénitier et Pinctada Maxima, **caractérisé en ce qu'**il consiste en l'hydrolyse à température inférieure à 5°C sous l'action de l'acide acétique de poudre de coquille externe et interne obtenue à partir de fragments de coquilles découpés, associée à la chromatographie et l'electrophorèse, ainsi qu'à l'action de réactifs colorés et de réactions enzymatiques.

2. Procédé selon la revendication 1, **caractérisé en ce que**, les fragments de coquille sont lavés sous pression de 3 bars, traités aux ultra sons, sans détergent pendant 5 mn, décontaminés pendant 1 heure par immersion dans une solution de calbénium, séchés puis placés dans des bols en zirconium contenant 6 billes en zirconium de 20 mn de diamètre, et placés dans un broyeur planétaire tournant alternativement à une vitesse de 1500 tr/mn pendant 2 périodes de 3 mn jusqu'à obtention d'une poudre de granulométrie de 300 à 500 microns qui est ensuite placée dans un récipient inerte contenant 58% d'eau apyrogène, 13% de glace broyée fabriquée à partir d'eau apyrogène et 29% d'acide acétique à 80%.

3. Procédé selon les revendications 1 et 2, **caractérisé en ce que** le mélange est agité par un agitateur, maintenu à un pH inférieur à 4,5 par un pH mètre couplé à une électrovanne, à une température constante inférieure à 5°C et plus souvent voisine de 0°C grâce à un circuit fermé dans lequel circule du polyéthylène glycol, la solution étant filtrée sur un tamis et centrifugée à une force de 18000 G. Le culot ainsi obtenu est lavé dans l'acide acétique à 5% puis centrifugé à nouveau à une force de 18000 G avant d'être repris dans de l'eau apyrogène à un pH 7 obtenu par adjonction de soude 2,5 N et refroidi par le même dispositif réfrigérant. La solution est ensuite agitée pendant 1 heure, puis centrifugé à nouveau à 18 000 G.

4. Procédé selon les revendications 1 à 3, **caractérisé en ce que** le culot est repris dans de l'eau apyrogène sous agitation constante, centrifugé à 18000 G, puis séché par lyophilisation à froid et broyé à une granulométrie de 0,2 à 0,5 microns.

5. Procédé selon les revendications 1 à 4, **caractérisé en ce que** les sumageants récupérés sont filtrés sur filtre en tissu, puis dessalés par passage sous pression de 9 bars, sur une batterie de 17 cassettes d'osmose tangentielle de 1 kg Dalton et de 0,5 $m^2$ chacune montées en série, puis dilués et filtrés à 6 reprises consécutives avec de l'eau apyrogène.

6. Procédé selon les revendications 1 à 5, **caractérisé en ce que**, au cours de l'avant dernière dilution le pH de la solution est amené à pH 7 par adjonction de soude 2,5N, concentrée au Rotavapor sous vide à moins 40°C lyophilisée à froid, l'extrait ainsi obtenu étant broyé au mortier à une granulométrie de 0,2 à 0,5 microns.

7. Procédé selon les revendications 1 à 6, **caractérisé en ce que** les protéines, les aminoacides, les glycoprotéines, les glycosaminoglycanes et les protéoglycanes extraits de la coquille de Tridacne Bénitier et de Pinctada Maxima, sont séparés par chromatographie et électrophorèse et identifiés par des réactifs colorés et des réactions enzymatiques spécifiques.

**Patentansprüche**

1. Verfahren zur Extraktion, Abtrennung und zum Nachweis von Wirkstoffen: Proteinen, Glykoproteinen, Glykosaminoglykanen und Proteoglykanen, welche in der Muschelschale von Tridacne Bénitier und Pinctada Maxima enthalten sind, **dadurch gekennzeichnet, dass** es aus der Hydrolyse bei einer Temperatur unterhalb von 5 °C unter Einwirkung von Essigsäure von Pulver äußerer und innerer Muschelschalen, das aus Bruchstücken zerstückelter Muschelschalen gewonnen wird, verbunden mit Chromatographie und Elektrophorese sowie der Einwirkung von Farbreagenzien und enzymatischer Reaktionen besteht.

2. Verfahren nach Anspruch 1, **dadurch gekennzeichnet, dass** die Bruchstücke der Muschelschale unter einem Druck von 3 bar gewaschen, 5 Minuten lang ohne Detergens ultraschallbehandelt, eine Stunde lang durch Eintauchen in eine Calbeniumlösung dekontaminiert, getrocknet, anschließend in Zirkoniumschalen, die 6 Zirkoniumkugeln von 20 mm Durchmesser enthalten, überführt und in ein Planetenmahlwerk verbracht werden, welches sich nacheinander in 2 Perioden von 3 min mit einer Geschwindigkeit von 1500 Upm dreht, bis ein Pulver einer Korngröße von 300 bis 500 Mikron erhalten wird, das darauf in einen inerten Behälter, der 58 % pyrogenfreies Wasser, 13 % aus apyrogenem Wasser hergestelltes zerstoßenes Eis und 29 % 80%ige Essigsäure enthält.

3. Verfahren nach Anspruch 1 und 2, **dadurch gekennzeichnet, dass** die Mischung in einem Rührer gerührt wird, der durch ein an ein Magnetventil gekoppeltes pH-Meter bei einem pH unterhalb von 4,5 und mittels eines geschlossenen Kreislaufs, in dem Polyethylenglykol zirkuliert, bei einer konstanten Temperatur unterhalb von 5 °C und zumeist in der Nähe von 0 °C eingestellt wird, wobei die Lösung durch ein Sieb filtriert und mit einer Kraft von 18.000 g zentrifugiert wird. Das so erhaltene Pellet wird in 5%iger Essigsäure gewaschen, anschließend erneut mit einer Kraft von 18.000 g zentrifugiert, bevor es in pyrogenfreiem Wasser mit pH 7, der durch Zugabe von 2,5 N Soda erhalten wird, aufgenommen und in derselben gekühlten Vorrichtung abgekühlt wird. Die Lösung wird darauf eine Stunde lang geschüttelt, dann erneut bei 18.000 g zentrifugiert.

4. Verfahren nach den Ansprüchen 1 bis 3, **dadurch gekennzeichnet, dass** das Pellet unter gleichmäßigem Schütteln in pyrogenfreiem Wasser aufgenommen wird, bei 18.000 g zentrifugiert, dann gefriergetrocknet und zu einer Korngröße von 0,2 bis 0,5 Mikron zermahlen wird.

5. Verfahren nach den Ansprüchen 1 bis 4, **dadurch gekennzeichnet, dass** der gewonnene Überstand über Gewebefilter filtriert wird, dann mittels Durchfließen einer Anordnung von 17 Kassetten tangentieller Osmose von 1 kg Dalton und 0,5 $m^2$, wobei jede in Serie geschaltet ist, unter einem Druck von 9 bar entsalzt, daraufhin durch 6-maliges aufeinander folgendes Aufnehmen in pyrogenfreiem Wasser verdünnt und filtriert wird.

6. Verfahren nach den Ansprüchen 1 bis 5, **dadurch gekennzeichnet, dass** im Laufe der vorletzten Verdünnung der pH der Lösung durch Zugabe von 2,5 N Soda auf pH 7 eingestellt wird, die Lösung in einem Rotavapor im Vakuum bei wenigstens 40 °C eingeengt, gefriergetrocknet und der so erhaltene Extrakt im Mörser auf eine Korngröße von 0,2 bis 0,5 Mikron zermahlen wird.

7. Verfahren nach den Ansprüchen 1 bis 6, **dadurch gekennzeichnet, dass** die aus der Muschelschale von Tridacne Bénitier und Pinctada Maxima extrahierten Proteine, die Aminosäuren, die Glykoproteine, die Glykoaminoglykane und die Proteoglykane durch Chromatographie und Elektrophorese aufgetrennt und durch Farbreagenzien und spezifische enzymatische Reaktionen nachgewiesen werden.

**Claims**

1. Method of extracting, separating and identifying the active constituents: proteins, glycoproteins, glycosaminoglycans and proteoglycans, which are contained in the Tridacne Bénitier and Pinctada Maxima shell, **characterised in that** it comprises the hydrolysis of external and internal shell powder, obtained from cut-up fragments of shell, at a temperature of less than 5° C under the action of acetic acid, associated with chromatography and electrophoresis, as well as with the action of coloured reagents and enzymatic reactions.

2. Method according to claim 1, **characterised in that** the fragments of shell are washed at a pressure of 3 bars, treated with ultrasounds, without any detergent for 5 mn, decontaminated for 1 hour by immersion in a calbenium solution, dried then placed in zirconium bowls containing 6 zirconium balls with a diameter of 20 mm, and placed in a planetary crusher rotating alternately at a speed of 1500 rpm for 2 periods of 3 mn until a powder with a

granulometry of between 300 and 500 microns is obtained, which powder is then placed in an inert vessel containing 58 % apyrogenic water, 13 % crushed ice produced from apyrogenic water and 29 % of 80 % acetic acid.

**3.** Method according to claims 1 and 2, **characterised in that** the mixture is agitated by an agitator, maintained at a pH lower than 4.5 by a pH measurer connected to an electrovalve, at a constant temperature of less than 5° C and more often close to 0° C by means of a closed circuit in which glycol polyethylene circulates, the solution being filtered through a sieve and centrifuged at a force of 18000 G. The residue, thus obtained, is washed in 5 % acetic acid, then centrifuged again at a force of 18000 G, prior to being placed again in apyrogenic water with a pH of 7, obtained by the addition of 2.5 N soda and cooled by the same cooling device. The solution is then agitated for 1 hour, then centrifuged again at 18000 G.

**4.** Method according to claims 1 to 3, **characterised in that** the residue is placed again in apyrogenic water under constant agitation, centrifuged at 18000 G, then dried by lyophilisation in the cold state and crushed to a granulometry of between 0.2 and 0.5 microns.

**5.** Method according to claims 1 to 4, **characterised in that** the recovered supernatants are filtered through a fabric filter, then desalinated by being passed, at a pressure of 9 bars, through a set of 17 tangential osmosis cassettes of 1 kg Dalton and 0.5 $m^2$, each mounted in series, then said supernatants are diluted and filtered 6 consecutive times with apyrogenic water.

**6.** Method according to claims 1 to 5, **characterised in that**, during the course of the penultimate dilution, the pH of the solution is brought to a pH of 7 by the addition of 2.5 N soda, which has been concentrated in the Rotavapor in a vacuum at less than 40° C and lyophilised in the cold state, the extract thus obtained being crushed in the mortar to a granulometry of between 0.2 and 0.5 microns.

**7.** Method according to claims 1 to 6, **characterised in that** the proteins, the amino-acids, the glycoproteins, the glycosaminoglycans and the proteoglycans, extracted from the Tridacne Bénitier and Pinctada Maxima shell, are separated by chromatography and electrophoresis and identified by coloured reagents and specific enzymatic reactions.